(19)

**Europäisches Patentamt**
**European Patent Office**
**Office européen des brevets**

(11)  **EP 2 755 504 B1**

(12)  **EUROPEAN PATENT SPECIFICATION**

(45) Date of publication and mention
of the grant of the patent:
**18.03.2015 Bulletin 2015/12**

(21) Application number: **12745894.1**

(22) Date of filing: **16.07.2012**

(51) Int Cl.:
*A23L 1/22* *(2006.01)*      *G01N 33/00* *(2006.01)*

(86) International application number:
**PCT/GB2012/051691**

(87) International publication number:
**WO 2013/038139 (21.03.2013 Gazette 2013/12)**

(54) **A METHOD OF CREATING FLAVOUR COMBINATIONS AND FLAVOURED PRODUCTS**

VERFAHREN ZUR ERZEUGUNG VON AROMAKOMBINATIONEN UND AROMATISIERTEN
PRODUKTEN

PROCÉDÉ DE CRÉATION D'ASSOCIATIONS D'ARÔMES ET DE PRODUITS AROMATISÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.09.2011 GB 201116003**

(43) Date of publication of application:
**23.07.2014 Bulletin 2014/30**

(73) Proprietor: **Zendegii Ltd**
**London Greater London W10 5QZ (GB)**

(72) Inventors:
• **EZAZ-NIKPAY, Khosro**
**London**
**Greater London SW13 0JS (GB)**

• **KOHN, Daniel**
**London**
**Greater London SW6 5RD (GB)**

(74) Representative: **Cookson, Barbara Elizabeth et al**
**Filemot Technology Law Ltd.,**
**25 Souhampton Buildings**
**London WC2A 1AL (GB)**

(56) References cited:
**WO-A1-2005/078433      WO-A2-2005/096842**
**GB-A- 1 348 869        US-A- 4 219 579**
**US-A- 4 990 354        US-A1- 2002 076 473**

## Description

## Technical Field

The present invention relates to a method of developing flavour combinations and products made with those flavour combinations.

## Background Art

The present invention is concerned with flavouring products by adding flavouring components into a product platform.

When designing novel tastes, typical methodologies involve the use of experience-based taste design by an expert, innovating around known successful flavour combinations, or randomly combining previously unexplored tastes. The disadvantage of these methods is a very long development time, a low rate of discovery of completely new flavour combinations, and products that have a very low margin of error in component concentration and variability.

Another method involves the unique combination of existing flavours. It is generally accepted that a more complex (multiple component) olfactory (i.e. flavour) experience is favourable in all categories (particularly wine and perfume). This method has the advantage that a great number of novel flavour and material attribute combinations can be developed from relatively few ingredients. One disadvantage of this method is that it generally involves time and labour consuming research and taste trials to ascertain exact product formulations and to determine which of the novel combinations are desirable and which are not. The fundamental and significant disadvantage of this method is that a thorough exploration of the vast number of combinations of components and concentrations of said components is prohibitively time and resource consuming.

Another method involves the application of a flavour source from one food where it is traditionally used into another food, where it is not. Product formulation using this method is facilitated because the material attributes of the flavour components are already known, which is advantageous. Further, the flavour itself is also known in the market and associated with known products, which may also be advantageous. An example of a method for selecting flavours that are relevant to a particular demographic group is described in WO 2005/096842 A (FRITO-LAY NORTH AMERICA, INC). 2005-10-20.

PTL 0002: GB 1348869 A (NESTLE SA). 1974-03-27. describes a method of developing a black tea product flavoured with an aromatic fruit extract. A testing method is described for establishing a perception threshold as the lowest concentration of fruit at which 7 of 35 testers detected the aroma. The black tea beverage composition is then established with a concentration below the perception threshold but sufficient to enhance the taste and aroma of the beverage. While one or more fruit extracts are suggested, it is merely taught to use additions that do not exceed any of the perception thresholds.

## Disclosure of Invention

The present invention provides a method of developing a flavoured product comprising a platform containing at least two added flavour components (CI=2) comprising the steps of:

(a) titrating each flavour component into the platform by adding increasing quantities of that flavour component to the platform and evaluating a human response thereto following each increment in order to identify a first concentration (DC) of that flavour component within the platform at which the presence of that component can be detected; and a second concentration of that flavour component within the platform at which that flavour component can be identified (IC);
(b) titrating a primary flavour component relative to an adjusted platform containing a concentration of each secondary component, which is present between its first and second concentrations in order to determine shifted first and second concentrations of the primary component relative to the adjusted platform; and then either
(c) using a concentration of that primary flavour component in the adjusted platform which is between the shifted first and second concentrations for the case; or, if further secondary flavour components are required,
(d) repeating from step (b) for an adjusted platform containing a mixture containing a further flavour component.

Preferably the method adds multiple flavour components each present between the DC and IC (referred to herein as the subtle space) enabling the creation of a complex flavour where all the concentrations are at below identification level producing a more differentiated olfactory response to the different flavours as they slowly disappear from the olfactory receptors. This is the same effect as a progression from bouquet to finish in a good wine.

The method can also be used to create a complex background and still allow a particular flavour to be embedded at above identification level within that background, thus making that flavour more interesting to the consumer. In this method it is possible to round out the flavour of a product that has an identifiable flavour, whether it be a single dominant flavour such as orange juice or a group of dominant flavours such as pineapple and coconut.

The invention includes a product designed using said methods, as described in claim 7, and in particular, such a product incorporating a complex flavour designed with each flavour component present in a concentration in which each component can be detected but not identified. Such a product prevents bad flavour interactions and produces a desirable flavour output even when the input flavour components are unexpected and unrelated. There is no limitation on the number of flavour components that can be incorporated.

Other preferred aspects of the invention are set out in the appended claims.

## Advantages of the Invention

Particular advantages obtained using the methods of the invention includes:

- speed of product development and market testing which reduces the time and cost of development
- a higher degree of freedom in the concentration of ingredients that may be necessary to compensate for cost and seasonality factors due to the fact that within the subtle space there is an inability to identify any of the ingredients; and
- the possibility of placing one or more ingredients at above identification limits within a background of a complex flavour thus creating a complex identifiable taste which, for example, will make a flavour based beverage taste more natural.

## Brief Description of Drawings

In order that the invention may be well understood, some embodiments thereof, will now be described, by way of example only, with reference to the accompanying diagrammatic drawings, in which:

Figure 1 is a representation of a three-dimensional flavour design space (subtle space); and
Figure 2 is an example illustrating exemplary titration curves for flavour components and how they shift as more ingredients are added to the mixture and the asymptotic behaviour as the complexity of the flavour (number of flavour components) is increased to larger numbers; and
Figure 3 is a representation of the subtle space as a function of the number of flavour components in a mixture.

## Description of a Preferred Embodiment

The present invention is a general method for the targeted search of desirable flavour combinations for foods and beverages. For the purposes of this embodiment, a smoothie based on a platform of apple juice will be described. It will be appreciated that the method of the invention can be used with any suitable food or beverage platform susceptible to the addition of flavour such as milk, water, cookie dough or chocolate.

The first step is the specification of an objective function to describe the key targeted attributes of the product. In this embodiment the attribute is complexity of flavour and the inability of tasters to identify one or more individual component flavour ingredients.

The method is computer implemented by the use of database structures to store a select number of parameters for a wide range of candidate flavour components. These parameters are chosen in order to facilitate the description of the salient attributes of the components. These parameters may also be chosen in order to facilitate the description of the components according to the objective function. A collection of parameters may be referred to as a vector for that flavour component.

## Dependent Parameters

Some parameters relate to human taste and olfactory response to a flavour component and are dependent on the platform and the presence of other flavour components. One such parameter is the minimum concentration or the average of the minimum concentration for a set of human tasters, of a component in a platform such that the component perceptibly alters the product flavour. This parameter is referred to herein as the Detection Concentration (DC) for a specific component. Another such parameter is the concentration or average concentration of a component such that the component is identifiably present to a taster who knows in advance that the component is present. This parameter is referred to as the Anticipation Concentration (AC) for a specific component. Another such parameter is the concentration or average concentration of a component such that the component is identifiably present to a taster who is familiar with the flavour of that particular component but does not know in advance that it is present. This parameter may be referred to as the Identification Concentration (IC) for a specific component. Another such parameter is the concentration or average concentration of a component such that the component is dominating or masking all of the other components present in the product. This parameter is referred to as the Saturation Concentration (SC) for a specific component.

These dependent parameters such as DC and IC are also dependent on other factors relevant to a product including the pH, temperature, the number of other flavours present, salt (Na+, K+, etc.), sugar (fructose, sucrose, glucose, etc.), and sweeteners (saccharin, aspartame, cyclamate, etc). They may also vary in dependence on the sourness, bitterness, and umami; the other tongue-based (as opposed to nose-based) flavour components.

Measurement of these dependent parameters may be carried out using human tasting panels or a suitably calibrated detection device or artificial nose which measures the presence of individual molecules or groups of molecule concentrations within the mixture. It is then possible to develop a database which records this knowledge, which will be particularly valuable in real world flavour design because it allows researchers to vary temperature, pH, sweetness, etc. in a complex product formula with a complete knowledge of how it will alter flavour subtleties.

## Independent Parameters

Parameters that relate to taste and olfaction include the basic taste bud taste dimensions of temperature, sweetness, pH, salinity, bitterness, and umami as well as olfactory responses to the flavour component.

Parameters may also be stored in the flavour component vector in the database to indicate whether the flavour component is compatible or incompatible with a variety of potential platforms. This information can be stored in inclusion and exclusion matrices for the platform as discussed further in the section on Platforms below.

## Objective Function of the Product

One measure of desirability of a food or beverage is its complexity and / or the inability of consumers to identify particular or dominant flavours. In this embodiment of the present invention, the objective function is both complexity of flavour and inability of tasters to identify individual flavours. There are many measures of complexity of flavour, one of which is number of flavour components. In the present example, complexity may be achieved by adding some number of flavour components e.g. between 1 and 10 to the platform. Another measure of complexity is the subtlety or inability of tasters to detect the presence of single ingredients. Likewise, there are many ways to achieve a mixture of flavours such that none of the component flavours are individually identifiable. In the present example, this may be achieved by adding components into the mixture at concentrations which are greater than their DC and less than their IC for any mixture of complexity index CI.

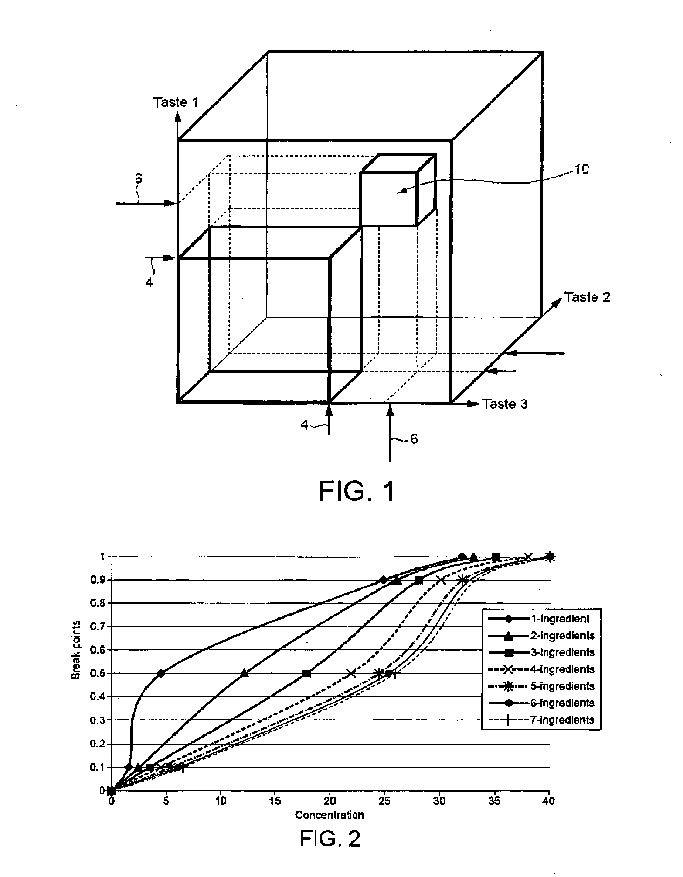

Figure 1 shows diagrammatically how for three ingredients the determination of the DC, 4 and IC, 6 dramatically reduces a subtle or design space 10 of the possible concentrations of the ingredients that will satisfy the objective criteria of inability of tasters to identify the presence of single ingredients while ensuring that the presence of the ingredient is detectable. The CI=3 complexity index has been illustrated for simpler visualisation in 3D. It will be appreciated that the same design space definition in which a subtle flavour that contains many components that can be detected but not identified is possible for any larger CI.

Given any mixture of M components, it is desirable to be able to quickly estimate a reasonable starting concentration value for each component prior to optimizing mixture concentrations for production. Further, it is desirable that the final production mixture not be sensitive to small fluctuations in concentrations of the flavour components. The methodology disclosed in the present description provides both desirable attributes. Regarding the first advantage, the method provides for a range for each component concentration which will impact the flavour of the mixture but which will not be identifiable given the existing set of components. This is the concentration range between the DC and IC at any CI. Regarding the second advantage, the method provides a specification of the mid-point between the DC (the limit below which the flavour component will be undetectable) and the IC (the limit above which the component will be identifiable). By using the midpoint concentration between the DC and IC a random variation from the starting concentration in either direction is protected from producing an undesirable mixture by either dropping below the DC or going above the IC. The midpoint is also guaranteed to lie in the unidentifiable concentration range. The midpoint is also a convenient starting point for further search to optimize component concentrations. The midpoint is also convenient if the exact temperature, pH, or other independent variable values that will describe the target product are unknown. The use of the DC/IC range and midpoint concentration is also a convenient starting concentration for the blending of known component mixtures, which allows for the creation of even higher complexity mixtures.

As data is developed on the knowledge of the shift of IC/DC with each added component, rule-based determination of the exact taste behaviour of any one component in a mixture of any number of other components can be employed. This information can be stored in a database of combinatorial rules, information and categorisation of combinations of ingredients within set parameters.

It is also found that as M (the number of flavour components) increases, the shift for an additional component reduces so that it is possible to record the large M value of the DC and IC as constant values in the database. These constant values can be used when making a flavoured product with high complexity of, for example more than 5 ingredients, preferably more than 7 ingredients.

**10 Ingredient Smoothie Example**

The product or environment parameters are platform, the inability of tasters to identify one or more individual component flavour ingredients, and CI. In this example, the platform is apple juice. The CI or complexity index is chosen as 10. The specific flavour components are chosen according to a product specification to deliver the required properties of the product as determined by the independent parameters of the flavour components used. Each candidate component is checked against the inclusion and exclusion matrix for apple juice.

The dependent parameters which need to be measured in order to establish a formulation in which tasters are unable to identify one or more individual component flavour ingredients of the product are DC, IC, and SC. A DC, IC, and SC are evaluated for a fixed platform value (apple juice) while increasing the CI from 1 (just mango puree in apple juice) to 10 (mango puree plus 9 other flavour constituents). Mango puree is referred to as the primary component because it is the component whose concentration is changed in the titration experiments used to determine the dependent parameters. Each of the 9 additional flavour components are referred to as a secondary component. The secondary ingredients or components include a variety of juices, purees, flavour extractions, and artificial flavours.

First we evaluate the DC, the IC, and the SC for mango puree (the primary component in this example) in apple juice (the platform in this example) alone. This evaluation is also carried out alone for each of the secondary components. This basic data may be found from an existing database or, if being evaluated for the first time, will be stored in the database for subsequent reuse. Ideally a record of the tasting panel used and any relevant demographic data will be stored with the data.

The method used to evaluate the DC, IC, and SC for the primary component alone (CI=1) is to perform a "titration experiment" by adding quantities of the primary component in small increments to the platform, performing subject taste evaluations against platform solutions alone. Increments of primary component are added until such a concentration is reached that the subjects are aware of a difference of flavour with the reference base solution. Likewise, increments may be added and compared to reference solutions until the IC and SC are determined. In the case of compound mixtures, where CI=2 or more, a representative or useful value of the concentration of the secondary components must be chosen. Many different values may be useful. By way of example, the secondary components may each be added in at a concentration which is half way between their single component DC and their single component IC. Evaluation of the two component DC, IC, and SC for the primary component may then proceed by adding the primary component in small increments to the platform/ secondary component mixture until the DC is determined. Likewise, additional primary component is added until the IC and SC are determined for these compound mixtures.

It is convenient to represent the measured DC, IC and SC parameters relative to the concentration as a series of shifting curves described herein as "titration curves" as illustrated in Figure 2. Each curve represents a best fit curve joining up the origin point of a horizontal axis representing concentration of the flavour component and a vertical (y) detection axis calibrated from 0 to 1, where the DC, IC and SC points are represented as y=0.1 0.5 and 0.9 respectively. This is an arbitrary assignment of value on the detection/y axis which has been found to produce a helpful visualisation of the titration curve.

Ideally to test a specific product with a CI of 10, the evaluation should be carried out with each of the next 9 ingredients and with the ingredients added in all possible orders. Even if we discount the order of addition, the number of unique combinations of N items chosen M at a time is N!/[M!*(N-M)!], which is N factorial divided by M factorial divided by N minus M factorial, where factorial is known to be the product of the number with all positive integers less than the number itself. In this example, CI=M+1, since the CI equals the total of the primary component (1) plus the number of secondary components (M). In this example N is the number of secondary components (9), and M is the number of secondary components that are added into the mixture. The total number of unique combinations for all of the values of M from 1 up to N is generally known from combinatorics and is equal to

$$\sum_{M=1}^{M=X} N! / [M! * (N-M)!]$$

In this example, where N=9 and M=1,2,...9, the total number of unique combinations is 512.

Evaluation of this number of combinations may be prohibitive. However, the number can be reduced by utilizing sampling methods to reduce experiment time. Many sampling methods generally known in the literature exist and may be used here, including those from the fields of combinatorial chemistry, population statistical sampling, drug trial methodologies, and the like. These methods include simple random sampling, systematic sampling, stratified sampling, probability proportional to size sampling, cluster/multistage sampling, matched random sampling, quota sampling, line intercept sampling, panel sampling, and event sampling. These and other methods for sampling from large populations exist and may be used within the present invention.

One preferred method of sampling of combinations utilizes a guided search. In the present example, it is known that certain secondary components will have a larger effect on the shift of the DC, IC, and SC in a positive direction while others will have either a minimal positive or a maximal negative effect on the shift of the DC, IC, and SC. The remaining secondary components will have a positive effect on the DC, IC, and SC that lie within the limits defined by these extrema. Knowing the range and bounds of the shift is important because it defines the limits of the possible shifts that could result from the mixture of any two of the selected ingredients. These extrema can be evaluated quickly by performing the CI=1 and then CI=2 experiments. In the CI=1 experiment, the baseline values of DC, IC, and SC are determined. In the CI=2 experiment, all 9 secondary components are evaluated and the maximal and minimal shifters are determined by differencing with the baseline values, that is a shift relative to the baseline CI = 1 value. A minimal shift in the CI=2 experiment indicates that the two flavour components have very little interaction (the primary is discernible despite the presence of the secondary) and a maximal shift in the CI =2 experiment indicates two flavour components may be very similar and it is difficult for the taster to distinguish them, or that one flavour simply masks the presence of the other. Based on the DC, IC, and SC shift results of the CI=2 experiments, the secondary components can be ordered into an array which may be called the secondary component array (SCA), which orders the secondary components in terms of their effect on the DC, IC, and SC of the primary component. The ordering and quantification of the shift from least to most in the SCA allows prediction of the expected effects and effects of combinations and it sets bounds on the effects of the secondary components. This can significantly reduce the number of actual combinations to be tested.

The value of the SCA is that it can significantly reduce the time required to evaluate the magnitude of the shift in DC, IC, and SC between the primary component alone (CI=1) values and any other complex flavour combinations (where CI=3 or greater). Since the primary component is always added in these titration experiments until it becomes the dominant flavour (sometimes in combination with the platform, which may be quite flavourful) the order of the SCA elements is not expected to change as the value of CI in the experiments changes from CI=2 to 9 i.e., their dominant interaction is with the primary component. The following example illustrates how the guided search utilizes the SCA and reduces the number of tested component mixtures. The SCA may be denoted SCA (primary component) =[sca1, sca2, ...sca9]. The element sca1 indicates the ingredient which provides the least shift from the baseline value. The maximal and minimal shifters, sca9 and sca1, define the extrema of the CI=2 combinations. In particular, in the 2-component experiment, the component sca1/sca9 would be mixed with the primary component, minimally/maximally shifting the DC, IC, and SC of the primary component to the new 2-component extrema. Of the remaining components [sca2,...,sca9]/[sca1,...,sca8] not used in the 2-component extrema mixtures, the components sca2/sca8 would be the new remaining minimal and maximal shifters left in the 3-component mixture (i.e., where CI=3), creating the ordered array [sca2,...,sca8] of secondary components for the CI=3 experiment. In gauging the shift of the CI=3 titrations then it is not necessary to mix the primary component with each of the 9 secondary components and then test each of the remaining components to evaluate their shifts. Instead, in order to gauge the magnitude of the CI=3 shifts, it is only necessary to evaluate the magnitude of the mixtures (primary component + sca1 + sca2)/(primary component + sca9 + sca8) to determine the minimal/maximal shifts of any of the combinations. All other combinations would fall within these bounds. So, in the CI=3 case alone, the method allows effective evaluation of the shift of titration curve ranges by evaluating just two of the 36 available primary plus two component mixtures. The same logic applies to CI=4 through 9. In the CI=4 case, the two likely extrema may be determined by combining sca1, sca2, and sca3 to evaluate the lower limit of the effect on the primary component's DC, IC, and SC and by combining sca7, sca8, and sca9 to evaluate the upper limit of the effect on the primary component's DC, IC, and SC. This process may be repeated, performing two extrema evaluations per CI level from CI=3 to 9. The total number of experiments that are evaluated using this guided search would then be 1 (the baseline, CI =1 experiment) +9 (all nine secondary components mixed individually with the primary component where CI =2) +2*(9-3+1) (two more experiments for each CI = 3 to 9) =24. In this example then, the guided search requires just 24/512 the number of experiments, or approximately 1/20$^{th}$ the number of experiments to evaluate the limits of the secondary components' effect.

It is also possible that the guided search or other sampling methods are not desired, particularly where it is desired to know the exact DC, IC, and SC shift for some specific component mixtures or for each and every multiple component mixture exactly. Even in such cases, there are still significant time-saving benefits from the method since the knowledge of DC and IC provides component concentration ranges that significantly reduce the range of testable concentrations as illustrated in Figure 1.

It will be noted from Figure 2, that as the complexity index rises, the spread between the concentration values for the DC and IC increases. This is because the shift with increasing complexity index of DC (y =0.1) in the figure is much lower than the shift in the IC (y=0 .5 in the figure). This widens the tolerance of component flavour concentrations that result in acceptable product flavour. This feature is beneficial for quality control and minimisation of variability in final product or intermediate component ingredient flavours. By way of example, this feature can be beneficial for foods and beverages where one or more input components suffer from natural or seasonal or regional variability. This feature is advantageous because the resulting product flavour is less susceptible to variations in input variability provided that the flavour component is present within the concentration range between the DC and the IC. It is also helpful where one artificial flavour needs to be substituted with another one due to availability or cost saving measures.

It can also be seen that the shift is reducing with CI and, provided the ingredients are diverse, the shift beyond 5 ingredients tends to a constant value which can reliably be used when CI is in the range 10 favour components or more.

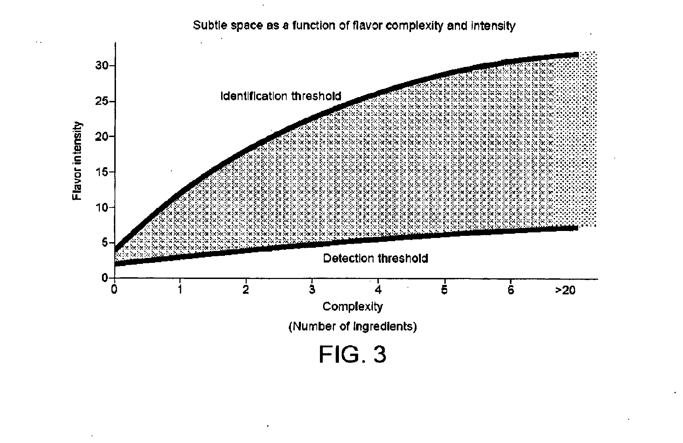

In Figure 3 "Flavour Intensity" is used as a proxy for flavour concentration because different categories of flavour providers may be added in very different amounts to achieve DC and IC e.g., concentrated artificial flavours are required in very low concentrations, distilled natural flavours may be used in higher concentrations, extracted natural flavours in higher concentrations, fruit concentrates in higher concentrations, and fruit juice in higher concentrations.

Platforms

In the example above, the platform was chosen to be apple juice. A platform can have one or multiple ingredients. Apple juice, for example, has multiple ingredients but the makeup can be considered standardised for apple juice from a specific source.

Further examples of liquid platforms include water, water with sugar and acid, carbonated water, dairy milk, low fat dairy milk, non-dairy milks such as soy milk, almond milk, hazelnut milk, fruit puree, ethanol, ethanol and aqueous mixtures, fermented e.g., beer, wines, liquors; aqueous extracts such as teas, coffee, infusions as well as non-aqueous extracted essences such bitters and liquors; and the like.

Examples of semi-liquid platforms include crushed ice, crushed frozen juice, crushed frozen milks and creams, yoghurt, frozen juice, frozen creams and milks, crushed frozen fruit, fruit purees and preserves, concentrated fruit and vegetables e.g., sauces, and the like.

Examples of solid and semi-solid platforms include roasted and unroasted cocoa as in the type used in the fabrication of confectionary chocolate and energy bars; grains as in the type used in the manufacture of muesli mixes and granola bars; pulses as in the type used in the manufacture of humus; and dough and flours as in the type used in the manufacture of baked goods.

The platform plays a significant role in the material aspects of the mixture. By way of example, milks may have a certain fat content that results in a thickness and/or mouth feel and smoothness of any mixture made using it as a platform. This effect results from colloidal and micelle content of the material. Colloidal fat emulsions however, can suffer from the introduction of low pH components, such as lemon or lime, which can either induce separation of the fat and water soluble layers or can lead to unpleasant experience for product tasters. Given this and other dominant effects of the platform with flavour components, it is advantageous to optionally have an inclusion and exclusion matrix of compatibilities of flavour components with each platform. The inclusion matrix lists the set of flavour components that are compatible with each platform. The exclusion matrix lists the set of flavour components that are not compatible with each platform. The inclusion matrix lists from the set of available flavour components those which pass a certain acceptable level of taste sensation when added to the platform alone in concentrations from DC to the IC. While there are potentially hundreds of individual flavour components which can be tested to complete the inclusion and exclusion matrix, the advantage is that once a component is included or excluded in the CI=1 experiment, it provides a predictive measure of the desirability of the component in all other mixtures where CI>1. This and other methods may be used to create inclusion and exclusion matrices. One further advantage of the inclusion matrix is that it may be used to suggest flavour combinations that are not obvious to experts in the field. By way of example, using this combinatorial technology, unusual mixtures of platform (e.g., 90% yogurt, 10% apple juice) and spices could generate positive responses in double blind screening of consumer evaluations. Likewise, a double blind evaluation of an inclusion matrix of fruits from a large selection of available fruits might lead to unexpected components with positive consumer response. When the spice and fruit inclusion matrices are combined and combination mixes are created, a wide variety of taste and olfactory complexity that might not otherwise have been generated will be created.

**Product parameters**

Parameters that relate to product environment include the number of components in the product. Such a parameter may be referred to as the Complexity Index (CI) of the environment of the mixture. Other parameters that relate to product environment include the temperature, pH, salt (Na+, K+, etc.), sugar (fructose, sucrose, glucose, etc.), and sweeteners (saccharin, aspartame, cyclamate, etc), the viscosity, the mouth feel and other somatosensory sensations, such as coolness, dryness, fattiness, heartiness (kokumi), numbness, and spiciness of the product. Another parameter that relates to product environment is the specification of the platform or predominant ingredient or ingredients of the mixture. Such a parameter may be referred to as the platform of the mixture. These and other parameters exist and may be added to a component vector as convenient.

**Claims**

1. A method of developing a flavoured product comprising a platform containing at least two added flavour components (CI=2) comprising the steps of:

   (a) titrating each flavour component into the platform by adding increasing quantities of that flavour component to the platform and evaluating a human response thereto following each increment in order to identify a first concentration (DC) of that flavour component within the platform at which the presence of that component can be detected; and a second concentration of that flavour component within the platform at which that flavour component can be identified (IC);
   (b) titrating a primary flavour component relative to an adjusted platform containing a concentration of each secondary component, which is present between its first and second concentrations in order to determine shifted first and second concentrations of the primary component relative to the adjusted platform; and then either
   (c) using a concentration of that primary flavour component in the adjusted platform which is between the shifted first and second concentrations for the case; or, if further secondary flavour components are required,
   (d) repeating from step (b) for an adjusted platform containing a mixture containing a further flavour component.

2. A method as claimed in claim 1, further comprising ordering the secondary components in dependence on the magnitude of shift in step (b) in order to reduce the repetitions in step (d).

3. A method as claimed in claim 2, wherein step (d) is only carried out for mixtures with the greatest and least shifting flavour components.

4. A method as claimed in any one of cairns 1 to 3, wherein each secondary flavour component is added to the platform in a concentration which is at the midpoint of the concentrations determined in step (a).

5. A method as claimed in claim 1, wherein the platform is or has a flavour added to it that is at a concentration where it can be detected.

6. A method as claimed in claim 2, wherein the detectable flavour is a combination of dominant flavours.

7. A product comprising at least 5 flavour components in a platform each present at a concentration between a first concentration (DC) of that flavour component measured within the platform in the presence of a large number of other diverse flavour components at which the presence of that component can be detected; and a second concentration of that flavour component within the platform measured in the presence of a large number of other diverse flavour components at which that flavour component can be identified (IC).

**Patentansprüche**

1. Verfahren zum Entwickeln eines aromatisierten Produkts, das ein Substrat umfasst, das mindestens zwei zusätzliche Aromabestandteile (CI (complexity index)=2) enthält, folgende Schritte umfassend:

   (a) Titrieren jedes einzelnen Aromabestandteils in das Substrat durch Hinzufügen zunehmender Mengen dieses Aromabestandteils zum Substrat und Auswerten einer menschlichen Reaktion darauf nach jeder Erhöhung, um eine erste Konzentration (detection concentration, DC) dieses Aromabestandteils innerhalb des Substrats, bei der das Vorhandensein dieses Bestandteils wahrgenommen werden kann, zu identifizieren, und eine zweite Konzentration dieses Aromabestandteils im Substrat, bei der das Vorhandensein dieses Bestandteils identifiziert werden kann (identification concentration, IC);
   (b) Titrieren eines Hauptaromabestandteils relativ zu einem eingestellten Substrat, das eine Konzentration jedes sekundären Bestandteils enthält, der zwischen seiner erster und zweiten Konzentration vorhanden ist, um die im Verhältnis zum korrigierten Substrat veränderte erste und zweite Konzentration des primären Bestandteils zu bestimmen, und dann entweder
   (c) Verwenden einer Konzentration dieses Hauptaromabestandteils in dem korrigierten Substrat, die zwischen der ersten und zweiten veränderten Konzentration in diesem Fall liegt, oder, falls weitere sekundäre Aromabestandteile erforderlich sind,
   (d) Wiederholen des Verfahrens ab Schritt (b) für ein eingestelltes Substrat, das eine Mischung mit einem weiteren Aromabestandteil enthält.

**2.** Verfahren nach Anspruch 1, ferner umfassend das Ordnen der sekundären Bestandteile in Abhängigkeit der Größe der Veränderung in Schritt (b), um die Wiederholungen in Schritt (d) zu verringern.

**3.** Verfahren nach Anspruch 2, wobei Schritt (d) nur für Mischungen durchgeführt wird, deren Aromabestandteile die größten oder geringsten Veränderungen aufweisen.

**4.** Verfahren nach einem der Ansprüche 1 bis 3, wobei jeder sekundäre Aromabestandteil dem Substrat in einer Konzentration zugefügt wird, die dem Mittelwert der in Schritt (a) bestimmten Konzentrationen entspricht.

**5.** Verfahren nach Anspruch 1, wobei das Substrat ein Aroma ist diesem ein solches hinzugefügt wird, das eine Konzentration aufweist, bei der es wahrnehmbar ist.

**6.** Verfahren nach Anspruch 2, wobei das wahrnehmbare Aroma eine Kombination aus dominanten Aromen ist.

**7.** Produkt mit mindestens 5 Aromabestandteilen in einer Substanz, von denen jeder in einer Konzentration zwischen einer ersten Konzentration (DC) dieses Aromabestandteils, gemessen im Substrat bei Vorhandensein einer großen Anzahl anderer unterschiedlicher Aromabestandteile, bei der das Vorhandensein dieses Bestandteils wahrgenommen werden kann; und einer zweiten Konzentration dieses Aromabestandteils im Substrat, gemessen bei Vorhandensein einer großen Anzahl anderer unterschiedlicher Aromabestandteile, bei der dieser Aromabestandteil wahrgenommen werden kann (IC), vorhanden ist.

**Revendications**

**1.** Procédé de mise au point d'un produit aromatisé comprenant une gamme contenant au moins deux composants d'arômes ajoutés (CI = 2) comprenant les étapes de :

(a) titrage de chaque composant d'arôme dans la gamme en ajoutant des quantités croissantes de ce composant d'arôme à la gamme et en évaluant une réponse humaine à celui-ci suite à chaque augmentation afin d'identifier une première concentration (DC) de ce composant d'arôme dans la gamme à laquelle la présence de ce composant peut être détectée ; et une seconde concentration de ce composant d'arôme dans la gamme à laquelle ce composant d'arôme peut être identifié (IC) ;
(b) titrage d'un composant d'arôme primaire par rapport à une gamme rectifiée contenant une concentration en chaque composant secondaire, qui est présent entre ses première et seconde concentrations afin de déterminer des première et seconde concentrations déplacées du composant primaire par rapport à la gamme rectifiée ; puis soit
(c) utilisation d'une concentration de ce composant d'arôme primaire dans la gamme rectifiée qui se situe entre les première et seconde concentrations déplacées pour le cas ; soit, si des composants d'arôme secondaires supplémentaires sont nécessaires,
(d) répétition de l'étape (b) pour une gamme rectifiée contenant un mélange contenant un composant d'arôme supplémentaire.

**2.** Procédé selon la revendication 1, comprenant en outre la commande des composants secondaires selon la grandeur de déplacement à l'étape (b) afin de réduire les répétitions à l'étape (d).

**3.** Procédé selon la revendication 2, dans lequel l'étape (d) est uniquement réalisée pour des mélanges avec les composants d'arôme aux déplacements le plus important et le moins important.

**4.** Procédé selon l'une quelconque des revendications 1 à 3, dans lequel chaque composant d'arôme secondaire est ajouté à la gamme dans une concentration qui est au point milieu des concentrations déterminées à l'étape (a).

**5.** Procédé selon la revendication 1, dans lequel la gamme est ou a un arôme qui lui est ajouté à une concentration où il peut être détecté.

**6.** Procédé selon la revendication 2, dans lequel l'arôme détectable est une combinaison d'arômes dominants.

**7.** Produit comprenant au moins 5 composants d'arôme dans une gamme, chacun étant présent à une concentration entre une première concentration (DC) de ce composant d'arôme mesurée dans la gamme en présence d'un grand nombre d'autres composants d'arôme divers à laquelle la présence de ce composant peut être détectée ; et une seconde concentration de ce composant d'arôme dans la gamme mesurée en présence d'un grand nombre d'autres composants d'arôme divers à laquelle ce composant d'arôme peut être identifié (IC).

FIG. 1

FIG. 2

Subtle space as a function of flavor complexity and intensity

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005096842 A
- GB 1348869 A